# EUROPÄISCHE PATENTSCHRIFT

(11) **EP 3 747 350 B1**
(45) Veröffentlichungstag und Bekanntmachung des Hinweises auf die Patenterteilung: **17.08.2022**
(21) Anmeldenummer: 20175838.0
(22) Anmeldetag: 20.05.2020
(51) Int. Cl.: A61B 1/06, F21V 29/60, F21V 29/503

(54) **VORRICHTUNG ZUR WÄRMEABLEITUNG AUS EINER ENDOSKOPISCHEN BELEUCHTUNGSEINRICHTUNG**
DEVICE FOR HEAT DISSIPATION FROM AN ENDOSCOPIC ILLUMINATION DEVICE
DISPOSITIF DE DISSIPATION THERMIQUE COMPRENANT UN DISPOSITIF D'ÉCLAIRAGE ENDOSCOPIQUE

(30) Priorität: 03.06.2019 DE 102019114885
(43) Veröffentlichungstag der Anmeldung: 09.12.2020
(73) Patentinhaber: Karl Storz SE & Co. KG, 78532 Tuttlingen (DE)
(72) Erfinder: Pascale, Costantino, 78532 Tuttlingen (DE)
(74) Vertreter: Witte, Weller & Partner Patentanwälte mbB

(56) Entgegenhaltungen:
- CN-A- 102 261 580
- DE-A1-102007 038 909
- DE-A1-102007 038 911
- DE-A1-102017 007 198
- US-A1- 2019 021 583

## Beschreibung

Die vorliegende Erfindung betrifft eine Vorrichtung für eine endoskopische Beleuchtungseinrichtung sowie eine endoskopische Beleuchtungseinrichtung mit der Vorrichtung.

Endoskope finden sowohl auf technischem Gebiet als auch auf medizinischem Gebiet Anwendung und eignen sich vor allem für die Inspektion von schwer zugänglichen und daher mit dem bloßen Auge nicht beobachtbaren Bereichen. Bspw. werden Endoskope im technischen Bereich für Arbeiten in Hohlräumen in Maschinen, Motoren, Turbinen und Reaktionsräumen usw. eingesetzt, die mit bloßem Auge nicht zu erkennen sind.

In der medizinischen Endoskopie werden Endoskope in der minimalinvasiven Chirurgie gegebenenfalls in Kombination mit chirurgischen Instrumenten zu Untersuchungszwecken oder für Operationen unter Sichtkontrolle oder auch zur Applikation von diagnostischem oder therapeutischem Licht verwendet.

Dabei weisen Endoskope zum einen ein Bildgebungssystem auf, das dazu dient, Beobachtungslicht aus dem Beobachtungsraum bzw. Operationsraum zu empfangen und Bildinformationen von distal nach proximal zu übertragen.

Das Bildgebungssystem kann ein optisches Bildübertragungssystem sein, das ein Objektiv im distalen Ende eines Schafts und ein sich daran anschließendes Linsensystem aufweist. Das Linsensystem kann bspw. ein Relais-System sein oder ein geordnetes Faserbündel und ein proximales Okular aufweist. Zudem ist es möglich, dass eine Kamera anschließbar ist.

Ein weiterer Bestandteil von Endoskopen ist ein Beleuchtungssystem, welches zur Übertragung von Licht von proximal nach distal dient, um den Beobachtungsraum bzw. Untersuchungsraum mit Licht auszuleuchten. Zur Beleuchtung werden herkömmlicherweise Hochleistungs-Leuchtdioden (LEDs) eingesetzt. Diese Technologie bietet insbesondere den Vorteil, dass sie im Vergleich zu der Xenon-Technologie eine nahezu gleichbleibende Lichtleistung über eine hohe Lebensdauer ermöglicht und zudem eine gleichbleibende Beleuchtungsqualität der Lichtquellen gewährleistet werden kann. Ein weiterer Vorteil der LED-Technologie ist, dass durch eine Kombination von LEDs unterschiedlicher Spektren erweiterte Diagnostiken wie z.B. ICG (Indocyaningrün) und PDD (photodynamische Diagnostik) realisierbar sind.

Damit bei der LED-Technologie das hohe Leistungsvermögen und die hohe Lebensdauer gewährleistet werden, ist ein ausreichend dimensioniertes Kühlmanagement maßgeblich, da die zum Einsatz kommenden LEDs trotz eines hohen Wirkungsgrads aufgrund ihrer hohen Lichtleistung weiterhin Wärmequellen darstellen.

Die von den LEDs bzw. Lichtquellen produzierte Abwärme kann bspw. in ein Gehäuse des Endoskops abgegeben werden (siehe z.B. US 8,696,554 B2). Diese Lösung der Wärmeabgabe kann jedoch zu einem Wärmestau führen, der ggf. zu einer Überhitzung der LEDs sowie deren Peripherie führen kann. Somit ist es vorteilhaft, wenn die entstehende Wärme von dem Entstehungsort hin zu einer Wärmesenke (bspw. in Form eines Kühlkörpers) geleitet wird. Dies wird im einfachsten Fall dadurch realisiert, dass Strömungspassagen innerhalb des Gehäuses des Endoskops vorgesehen sind, die bspw. initiiert durch einen Lüfter mit einem Luftstrom durchströmt werden (siehe US 5,099,399 A). Diese Lösung ist allerdings nachteilig, da durch die Strömungskanäle innerhalb des Gehäuses die Freiheit bei der konstruktiven Ausgestaltung desselben eingeschränkt ist.

Eine Möglichkeit zur Steigerung der Freiheit bei der konstruktiven Ausgestaltung und Wärmeableitleistung besteht darin, eine räumliche Trennung zwischen Wärmequelle und Wärmesenke durch den Einsatz sog. Heatpipes vorzunehmen. Diese Heatpipes, die auch Wärmeleitrohre genannte werden, ermöglichen bspw. eine strömungstechnisch optimale Positionierung der Wärmesenke innerhalb der endoskopischen Beleuchtungseinrichtung und dienen zum Wärmeaustausch zwischen der Wärmequelle und der Wärmesenke.

Die Anbindung von Heatpipes an die Wärmequellen erfolgt bspw. über Löten oder Schweißen. Zudem können die Heatpipes auch integral mit der Wärmequelle verbunden sein. Eine beispielhafte Wärmeableitung mittels Heatpipes kann der JP 2014-045820 A und der EP 1 731 862 A1 entnommen werden. Die JP 2014-045820 A und die EP 1 731 862 A1 zeigen jeweils eine Bauweise, bei der die Heatpipe integral mit der Wärmequelle verbunden ist.

DE 10 2017 007 198 A1 offenbart kontaminationsfrei kühlbare, umschlossene, bei Betrieb Wärme abgebende, elektrische und/oder elektronische Bauteile und Geräte.

DE 10 2007 038 911 A offenbart eine Kühlvorrichtung und Beleuchtungseinrichtung.

DE 10 2007 038 909 A1 offenbart ein Wärmeleitrohr und eine Anordnung mit Wärmeleitrohr.

CN 102 261 580 A offenbart eine LED-Vorrichtung mit einem Metallpodest, das eine erste und einer zweite Kontaktplatte aufweist zwischen denen Heatpipes angeordnet sind.

Es ist eine Aufgabe der vorliegenden Erfindung, eine Vorrichtung zur Wärmeableitung aus einer endoskopischen Beleuchtungseinrichtung derart weiterzuentwickeln, dass ein höheres Maß an Freiheit bei der konstruktiven Ausgestaltung und/oder eine Steigerung der Wärmeableitleistung gewährleistet werden kann.

Die Aufgabe wird durch eine Vorrichtung für eine endoskopische Beleuchtungseinrichtung nach Anspruch 1 gelöst.

Bedingt durch die Funktionsweise der Heatpipe tritt während der Wärmeabfuhr der durch die Wärmequelle erzeugten Wärme ein Temperaturgradient zwischen dem heißen Ende der Heatpipe, welches an der Wärmequelle angeordnet ist, und dem kalten Ende der Heatpipe, welches in der Wärmesenke angeordnet ist, auf. Dieser Temperaturgradient führt entlang der Heatpipe zu unterschiedlichen, materialbedingten Temperaturausdehnungen.

Die Erfinder haben erkannt, dass sich die entstehenden Eigenspannungen aufgrund der starren Befestigung der jeweiligen Enden der Heatpipe mit der Wärmequelle bzw. Wärmesenke auf andere Komponenten der endoskopischen Beleuchtungseinrichtung übertragen können. Dadurch kann es bspw. zur Genauigkeitsbeeinträchtigung bei der Bildaufnahme kommen.

Zudem wurde erkannt, dass an das Positionieren der Wärmequellen (insbesondere LEDs) relativ zu den Heatpipes aufgrund von Fertigungstoleranzen der Heatpipes hohe Anforderungen gestellt werden müssen. Auch ist das Positionieren mit einem hohen Justage- bzw. Montageaufwand verbunden, da die Anordnung der Heatpipes bspw. aufgrund einer vorbestimmten Lage und Ausrichtung des Bildgebungssystems, insbesondere des Linsensystems und des Objektivs (z.B. eines Glasstabes), beschränkt ist.

In diesem Zusammenhang haben die Erfinder erkannt,, dass bspw. eine parallele Wärmeabführung (ein geradliniger Verlauf der Heatpipes) nur schwer zu realisieren ist und die Heatpipes zur Unterbringung in dem Gehäuse der endoskopischen Beleuchtungseinrichtung in der Regel einem gekrümmten Kurvenverlauf folgen, durch den die Folgen eines Verziehens oder Verformens durch Eigenspannung verstärkt werden.

Einer der Vorteile der Ausgestaltung ist, dass die Heatpipe nicht wie im Stand der Technik üblich, fix mit der Wärmequelle bzw. dem Wärmeabfuhrelement der Wärmequelle verbunden ist, sondern reversibel lösbar an diesem montiert ist. Durch die Anordnung der Heatpipe zwischen dem Wärmeabfuhrelement und dem Klemmelement, d.h. also durch das Einklemmen bzw. Einspannen der Heatpipe, ist diese nicht fix mit ihrem einen Ende mit dem Wärmeabfuhrelement verbunden, sondern lediglich kraftschlüssig eingespannt. Dadurch werden insbesondere die zuvor genannten Eigenspannungen nichtmehr auf die die Heatpipe umgebenden Komponenten übertragen.

Durch die Aufnahme der Heatpipe zwischen dem Wärmeabfuhrelement und dem Klemmelement ist zudem die zur Wärmeübertragung zur Verfügung stehende Oberfläche vergrößert, da die Heatpipe in dem ersten Endbereich von dem Wärmeabfuhrelement und dem Klemmelement umschlossen ist. Das heißt, dass die gesamte Außenfläche des ersten Endbereichs der Heatpipe zur Wärmeübertragung zur Verfügung steht.

Zudem wird durch diese Anbringung der Heatpipe die Freiheit bei der konstruktiven Ausgestaltung maßgeblich erhöht, da sowohl das Wärmeabfuhrelement als auch das Klemmelement an einen jeweiligen Verlauf der Heatpipe angepasst werden kann. Somit ist eine modulare Anordnung der Heatpipe möglich. Zudem können bspw. die zuvor erwähnten Fertigungstoleranzen der Heatpipe im Gesamtsystem ausgeglichen werden, was sich wiederum in einer erhöhten Freiheit bei der konstruktiven Ausgestaltung widerspiegelt.

Die Ausgestaltung bietet insbesondere für den Retrofit, d.h. für das nachträgliche Verändern einzelner Komponenten, einen geeignete Möglichkeit, da der Klemmmechanismus reversibel lösbar ausgeführt ist. Dies führt dazu, dass die Heatpipe bspw. leicht ausgetauscht werden kann. In einigen Ausgestaltungen kann die Heatpipe auch mit dem Kühlkörper verlötet sein, wobei in einem solchen Fall bei einem Retrofit der Kühlkörper samt Heatpipe ersetzt werden kann.

Mit anderen Worten ist das Wärmeabfuhrelement also an der Wärmequelle, die bspw. Bestandteil einer Optikeinheit einer endoskopischen Beleuchtungseinrichtung ist, positionsgenau montiert. Die Anbindung der Heatpipe erfolgt anders als bei dem Stand der Technik allerdings nicht durch Löten oder Schweißen, also nicht durch ein formschlüssiges, nicht-reversibles Verfahren, sondern unter Verwendung des Klemmelements.

Unter den Begriffen "Wärmeabfuhrelement" und "Klemmelement" wird vorliegend ein vorzugsweise im Wesentlichen platten- bzw. quaderförmiger Körper, bspw. aus einem Metall oder einer Legierung verstanden. Der Körper weist vorzugsweise eine oder mehrere Ausnehmungen auf. Die Ausnehmungen sind vorzugsweise dafür ausgebildet, die Heatpipe zumindest teilweise aufzunehmen. Dabei kann bspw. ein stabförmiger Endabschnitt der Heatpipe anteilig in einer Ausnehmung des Wärmeabfuhrelements und anteilig in einer der Ausnehmung des Wärmeabfuhrelements der Form nach entsprechenden Ausnehmung des Klemmelements aufgenommen sein, so dass der Endabschnitt der Heatpipe von beiden Ausnehmungen vorzugsweise vollständig umfasst ist.

Unter dem Begriff "Heatpipe" wird vorliegend ein Wärmeleitrohr verstanden. Wärmeleitrohre umfassen dabei vorzugsweise metallene Gefäße länglicher Form, welche ein hermetisch gekapseltes Volumen aufweisen. Das Volumen ist mit einem Arbeitsmedium (z. B. Wasser oder Ammoniak) gefüllt, das das Volumen zu einem kleinen Teil in flüssigem, zu einem im Verhältnis größeren Teil im gasförmigen Zustand ausfüllt. Das Arbeitsmedium wird während der Verwendung des Wärmeleitrohres im Bereich der Wärmequelle verdampft. In dem Bereich der Wärmesenke kondensiert das Arbeitsmedium wieder und kehrt getrieben von Kapillarkräften zum wärmequelleseitigen Bereich des Wärmeleitrohres zurück.

Zudem kann unter dem Begriff "Heatpipe" auch ein Metallstab aus Vollmaterial bspw. mit rundem oder rechteckigem Querschnitt verstanden werden, bei dem der Wärmetransport durch Konduktion, d.h. durch die Übertragung kinetischer Energie zwischen benachbarten Atomen des Metallstabs ohne Materialtransport erfolgt.

Gemäß einer bevorzugten Ausgestaltung ist die Wärmequelle eine Lichtquelle.

Unter dem Begriff "Lichtquelle" wird vorliegend vorzugsweise eine Leuchtdiode (kurz: LED) verstanden. Grundsätzlich kann aber auch bspw. eine Xenon-Leuchte als Lichtquelle zum Einsatz kommen, wobei Leuchtdioden aufgrund ihrer im Verhältnis zu Xenon-Leuchten erhöhten Lebensdauer sowie aufgrund eines besseren Wirkungsgrads bevorzugt sind.

Gemäß einer weiteren bevorzugten Ausgestaltung ist das Klemmelement mit dem Wärmeabfuhrelement lösbar verschraubt oder verstiftet.

Diese Ausgestaltung hat insbesondere den Vorteil, dass das Klemmelement in einfacher Weise an dem Wärmeabfuhrelement montiert werden, jedoch auch in einfacher Weise wieder von diesem gelöst werden kann. Bspw. kann das Klemmelement über eine oder mehrere Schrauben oder Bolzen mit dem Wärmeabfuhrelement verbunden sein. Bei einem Verstiften (d.h. dem Einschieben von Bolzen zur Befestigung) ist es bevorzugt, wenn sog. Sicherungssplinte gegen ein Herausziehen der Splinte eingesetzt werden.

Gemäß einer weiteren bevorzugten Ausgestaltung sind die Heatpipe, das Wärmeabfuhrelement und das Klemmelement jeweils aus einem thermisch leitfähigen Werkstoff hergestellt.

Der wärmeleitfähige Werkstoff ist vorzugsweise derart ausgebildet, dass die an der Wärmequelle bzw. die durch die Leuchtdiode erzeugte Wärme vollständig von dem Wärmeabfuhrelement an die Heatpipe übertragen wird, ohne dass ein Wärmerückstau aufgrund von wärmeübertragungshemmenden Eigenschaften auftritt. Hierbei erfolgt die Wärmeübertragung zwischen dem Wärmeabfuhrelement und der Heatpipe vorzugsweise über eine gemeinsame Grenzfläche. Das heißt, dass zwischen dem Wärmeabfuhrelement und der Heatpipe vorzugsweise kein Luftspalt, der eine isolierende Wirkung für den Wärmetransport hat, existiert. In einer Ausgestaltung sind die Heatpipe und Wärmeabfuhrelement aus Kupfer, das Klemmelement aus Aluminium hergestellt. Es sind jedoch auch Ausgestaltungen denkbar, bei denen die Heatpipe, das Wärmeabfuhrelement und das Klemmelement vorzugsweise alle aus dem gleichen thermisch leitfähigen Werkstoff hergestellt sind.

Gemäß einer weiteren bevorzugten Ausgestaltung weist das thermisch leitfähige Material Aluminium oder Kupfer auf oder ist Aluminium oder Kupfer.

Vorliegend sind sowohl thermisch leitfähige Materialien umfasst, die lediglich bspw. als Legierungsbestandteil Kupfer und/oder Aluminium aufweisen, jedoch auch solche die aus reinem Kupfer oder Aluminium bestehen. Hierbei hängt es nicht davon ab, welchen Anteil an Kupfer und/oder Aluminium das thermisch leitfähige Material aufweist, solang eine Wärmeleitfähigkeit des thermisch leitfähige Materials gewährleistet ist, die im Wesentlichen (± 20 %) der Wärmeleifähigkeit von Kupfer oder Aluminium entspricht.

Gemäß einer weiteren Ausgestaltung ist zwischen einer Oberfläche des Wärmeabfuhrelements, einer Oberfläche des ersten Endbereichs der Heatpipe und einer Oberfläche des Klemmelements eine Wärmeleitpaste zur thermischen Kopplung aufgebracht.

Ein Vorteil der zusätzlich aufgebrachten Wärmeleitpaste ist, dass dadurch die thermische Leitfähigkeit im Bereich der Grenzschicht, das heißt, in dem Bereich in dem sich die jeweiligen Oberflächen des Wärmeabfuhrelements, des ersten Endbereichs der Heatpipe und des Klemmelements berühren, erhöht ist. Beispielsweise kann auch nur im Kontaktbereich der Oberfläche des Wärmeabfuhrelements und der Oberfläche des ersten Endbereichs der Heatpipe die Wärmeleitpaste aufgebracht sein, da in diesem Kontaktbereich der Großteil der Wärmeübertragung der abzuleitenden Wärme erfolgt. Anstelle von Wärmeleitpaste kann auch ein Wärmeleitpad eingesetzt werden. Vorzugsweise kann alternativ oder ergänzend Wärmeleitpaste bzw. ein Wärmeleitpad zwischen der Lichtquelle (bzw. LED) und dem Wärmeabfuhrelement angeordnet sein.

Unter der Oberfläche wird vorliegend bspw. eine Oberfläche einer Wand bzw. eines Wandabschnitts des Wärmeabfuhrelements, des ersten Endbereichs der Heatpipe und des Klemmelements verstanden.

Der erste Endbereich der Heatpipe ist vorzugsweise der Bereich, der in dem montierten Zustand zwischen dem Wärmeabfuhrelement und dem Klemmelement eingeklemmt ist.

In einer weiteren bevorzugten Ausgestaltung ist die zweite Wärmequelle eine zweite Lichtquelle. Für die zweite Lichtquelle gilt das oben, zu der ersten Lichtquelle Genannte entsprechend.

In einer weiteren bevorzugten Ausgestaltung weist die endoskopische Beleuchtungseinrichtung ferner einen Kühlkörper auf, der die Wärmesenke der Vorrichtung bildet und vorzugsweise eine Vielzahl von Kühlrippen aufweist. Zudem weist die endoskopische Beleuchtungseinrichtung einen Lüfter auf, der dafür ausgebildet ist, einen in Richtung zum oder in Gegenrichtung weg vom Kühlkörper weisenden Luftstrom zu erzwingen.

Unter dem Begriff "Kühlkörper" kann vorliegend bspw. ein blockartiger metallischer Körper verstanden werden, der derart geformt ist, dass seine wärmeabgebende Oberfläche im Vergleich zu einem durchgehenden metallischen Quader vergrößert ist. Die Vergrößerung der Oberfläche kann bspw. durch eine Vielzahl von Einkerbungen und oder Auswölbungen erreicht werden.

Der Lüfter bzw. das Gebläse ist vorzugsweise dafür ausgebildet, stehende Umgebungsluft durch eine Drehung von Rotorblättern derart zu beschleunigen, dass ein erzwungener Luftstrom entsteht, der vorzugsweise den Kühlkörper direkt anströmt oder die den Kühlkörper umgebende Luft direkt von diesem absaugt. Der Lüfter ist vorzugsweise an dem oder in der Nähe des Kühlkörpers angeordnet.

Es versteht sich, dass die vorstehend genannten und die nachstehend noch zu erläuternden Merkmale nicht nur einzeln, sondern auch in beliebiger Kombination untereinander oder in Alleinstellung verwendet werden können, ohne den Rahmen der vorliegenden Erfindung zu verlassen.

Ausführungsbeispiele einer endoskopischen Beleuchtungseinrichtung sind in den Zeichnungen dargestellt und werden in der nachfolgenden Beschreibung näher erläutert. Es zeigen:
- Fig. 1: eine Draufsicht einer endoskopischen Beleuchtungseinrichtung mit einem Ausführungsbeispiel der Vorrichtung;
- Fig. 2: eine perspektivische Ansicht des Ausführungsbeispiels der Vorrichtung;
- Fig. 3: eine Draufsicht der in Fig. 2 gezeigten Ansicht des ersten Ausführungsbeispiels;
- Fig. 4: eine perspektivische Ansicht eines zweiten Ausführungsbeispiels der Vorrichtung;
- Fig. 5: eine Explosionsansicht des zweiten Ausführungsbeispiels;
- Fig. 6: eine perspektivische Ansicht eines ersten Ausführungsbeispiels des Klemmelements;
- Fig. 7: eine perspektivische Ansicht eines zweiten Ausführungsbeispiels des Klemmelements;
- Fig. 8: eine perspektivische Ansicht von Heatpipes und Kühlkörper im verlöteten Zustand;
- Fig. 9: eine Schnittansicht der in Fig. 8 gezeigten perspektivischen Ansicht; und
- Fig. 10: eine Schnittansicht der erfindungsgemäßen Heatpipe(s) in eingespanntem Zustand.

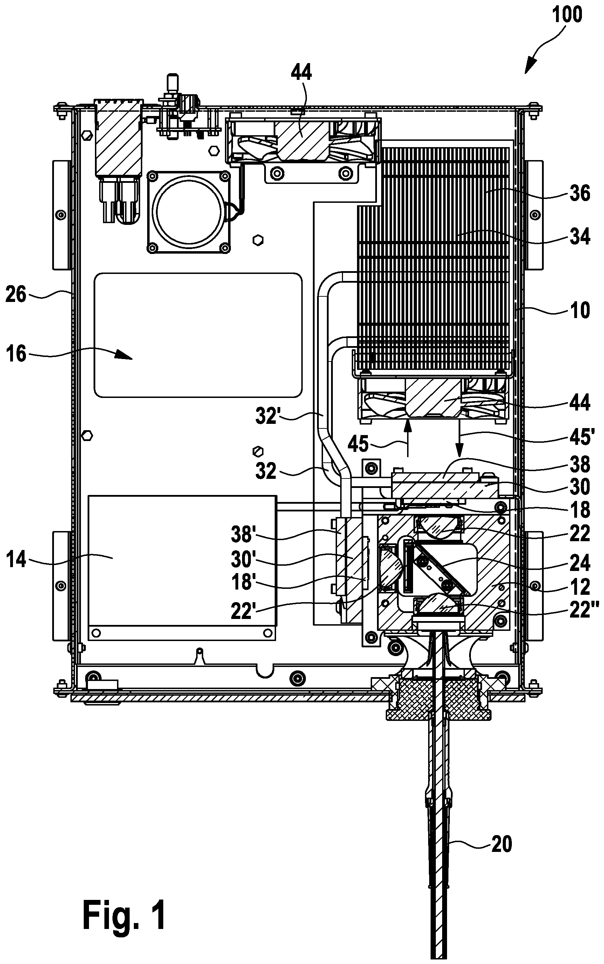

Alle folgenden Ausführungsbeispiele der Vorrichtung fallen nur dann unter die vorliegende Erfindung, wenn sie gemäß Anspruch 1 ein Wärmeabfuhrelement in L-Form aufweisen. Fig. 1 zeigt eine endoskopische Beleuchtungseinrichtung mit einem Ausführungsbeispiel der Vorrichtung. Die endoskopische Beleuchtungseinrichtung ist in ihrer Gesamtheit mit der Bezugsziffer 100, die Vorrichtung mit der Bezugsziffer 10 gekennzeichnet.

Die endoskopische Beleuchtungseinrichtung 100 weist in dem in Fig. 1 gezeigten Ausführungsbeispiel als Hauptkomponenten die Vorrichtung 10, eine Optikeinheit 12, eine Lichtquellen-Treiberplatine 14 und ein Netzteil 16 auf.

Eine Hauptplatine (auch Mainboard genannt) ist in der gezeigten Schnittansicht nicht dargestellt. Die Hauptplatine ist vorzugsweise oberhalb des Netzteils 16 angeordnet und kann vorzugsweise als Steuer- und Auswerteeinheit der endoskopischen Beleuchtungseinrichtung 100 angesehen werden. Das Netzteil 16 ist dafür ausgebildet, die endoskopische Beleuchtungseinrichtung 100 bzw. sämtliche Bestandteile dieser jeweils mit elektrischer Energie zu versorgen und weist bspw. eine Leistung von bis zu 150 W bei Konvektionskühlung, bis zu 250 W bei Konduktionskühlung und bis zu 550 W bei erzwungener Kühlung auf.

Die Optikeinheit 12 bildet den "Kern" der endoskopischen Beleuchtungsvorrichtung 100 und ist dafür ausgebildet, Licht, das von einer oder mehreren Lichtquellen 18, 18' emittiert wird, derart zu fokussieren, dass das Licht als konzentriertes Lichtstrahlbündel in einen Lichtleiter 20 eingeleitet wird. Vorliegend weist die Optikeinheit 12 einen Grundträger auf, der bspw. aus Aluminium gefertigt sein kann. Die erste und die zweite Lichtquelle 18, 18' sind vorliegend plattenförmige Leuchtdioden (bspw. in SMD-Bauweise). Die erste und zweite Lichtquelle 18, 18' sind orthogonal zueinander angeordnet. Die erste Lichtquelle 18 ist gegenüberliegend von den Lichtleiter 20 angeordnet, blickt diesem demnach entgegen.

Vor der ersten und der zweiten Lichtquelle 18, 18' sowie vor dem Lichtleiter 20 ist jeweils eine optische Linse 22 (bspw. eine Kollimator-Optik) angeordnet. Die Linsen 22 werden vorzugsweise jeweils durch eine Linsenhalterung gehalten. Die Linsen 22 sind dazu ausgebildet, das durch die Lichtquellen 18, 18' erzeugte Licht auszurichten bzw. zu kollimieren. Die vor die erste und die zweite Lichtquelle 18, 18' geschalteten Optiken 22, 22', 22" fokussieren das erzeugte Licht dabei auf ein Zentrum eines Strahlteilers 24. Der Strahlteiler 24 ist gegenüber der beiden Lichtquellen 18, 18' derart ausgerichtet, dass er zu jeder der beiden Lichtquellen 18, 18' eine 45° Stellung hat.

Der Strahlteiler 24 ist dafür ausgebildet, das Licht der ersten Lichtquelle 18 geradlinig, d.h. ohne Ablenkung, in Richtung des Lichtleiters 20 passieren zu lassen, wohingegen der Strahlteiler 24 das von der zweiten Lichtquelle 18' stammende und durch die Linse 22' kollimierte Licht um 90° in Richtung des Lichtleiters 20 ablenkt. Hierfür kann der Strahlteiler 24 bspw. eine teiltransmittive Beschichtung aufweisen. Das in den Lichtleiter 20 gelenkte Licht wird vor dem Eintritt in den Lichtleiter 20 nochmals durch die Linse 22" kollimiert. Das auf diese Weise durch die Optikeinheit 12 gebündelte Licht kann durch den Lichtleiter 20 an einen mit der endoskopischen Beleuchtungseinrichtung 100 zu beleuchtenden Einsatzort geleitet werden.

Zur Ansteuerung der Lichtquellen 18, 18' während des Betriebs wird die Hauptplatine vorzugsweise von Netzteil 16 mit elektrischer Energie versorgt. Ansteuersignale zum Ansteuern der Lichtquellen 18, 18' werden vorzugsweise von der Hauptplatine auf die jeweilige Lichtquellen-Treiberplatine übertragen. Die Lichtquellen-Treiberplatine(n) transformiert die Leistung zu der jeweiligen Lichtquelle 18, 18'. Obwohl als Lichtquellen 18, 18' vorliegend hochleistungsstarke Leuchtdioden mit hohem Wirkungsgrad zum Einsatz kommen, entsteht bei dem Betrieb der beiden Lichtquellen 18, 18' Abwärme, die bei einem Nichtableiten zu einem Wärmestau, bspw. in einem Gehäuse 26 der endoskopischen Beleuchtungseinheit 100, führen könnte. Die Lichtquellen 18, 18' sind demnach Wärmequellen 28, 28', wobei die erste Lichtquelle 18 eine erste Wärmequelle 28 repräsentiert und die zweite Lichtquelle 18' eine zweite Wärmequelle 28' repräsentiert. In anderen Ausgestaltungen ist auch nur eine Wärmequelle 28, 28' bzw. eine Lichtquelle 18, 18' vorhanden.

Zum Abführen der an den Lichtquellen 18, 18' entstehenden thermischen Energie (bzw. Abwärme) weist die Vorrichtung 10 ein Wärmeabfuhrelement 30 auf. Das Wärmeabfuhrelement 30 ist in einer direkten Peripherie der ersten Wärmequelle 28 und der zweiten Wärmequelle 28' angeordnet. Ein derartiges Wärmeabfuhrelement 30 weist eine L-Form auf und ist somit zur Abfuhr der thermischen Energie von sowohl der ersten als auch von der zweiten Wärmequelle 28, 28' ausgebildet.

Die durch das erste Wärmeabfuhrelement 30 abgeführte thermische Energie der ersten Wärmequelle 28 wird vorliegend über eine erste Heatpipe 32 an eine von der ersten Wärmequelle 28 beabstandete Wärmesenke 34 abgeführt. Vorliegend weist die Vorrichtung noch eine zweite Heatpipe 32' auf.

Die erste und zweite Heatpipe 32, 32' sind vorliegend rohrförmig oder stabförmig ausgeführt und unterscheiden sich in ihrer jeweiligen Form. Hierbei ist die Form, d.h. der Verlauf des Rohres, der jeweiligen Heatpipe 32, 32' an die Lage und Positionierung der Wärmeabfuhrelemente 30, 30' angepasst. Die Heatpipes 32,32' weisen bspw. mehrere geradlinig verlaufende Rohrabschnitte sowie mehrere Biegungen und/oder gebogene Rohrabschnitte auf.

Die erste Heatpipe 32 ist mittels eines ersten Klemmelements 38 derart auf dem ersten Wärmeabfuhrelement 30 reversibel lösbar montiert, dass ein erster Endbereich 40 der ersten Heatpipe 32 zwischen dem ersten Wärmeabfuhrelement 30 und dem ersten Klemmelement 38 gehalten ist. Die zweite Heatpipe 32' ist vorzugsweise mittels eines zweiten Klemmelements 38' derart auf dem zweiten Wärmeabfuhrelement 30' reversibel lösbar montiert, dass ein erster Endbereich 40' der zweiten Heatpipe 38' zwischen dem zweiten Wärmeabfuhrelement 30' und dem zweiten Klemmelement 38' gehalten ist. In einem Fall, in dem es lediglich ein Wärmeabfuhrelement 30, 30' gilt, sind vorzugsweise beide Klemmelemente 38, 38' auf dem einzigen Wärmeabfuhrelement 30, 30' montiert.

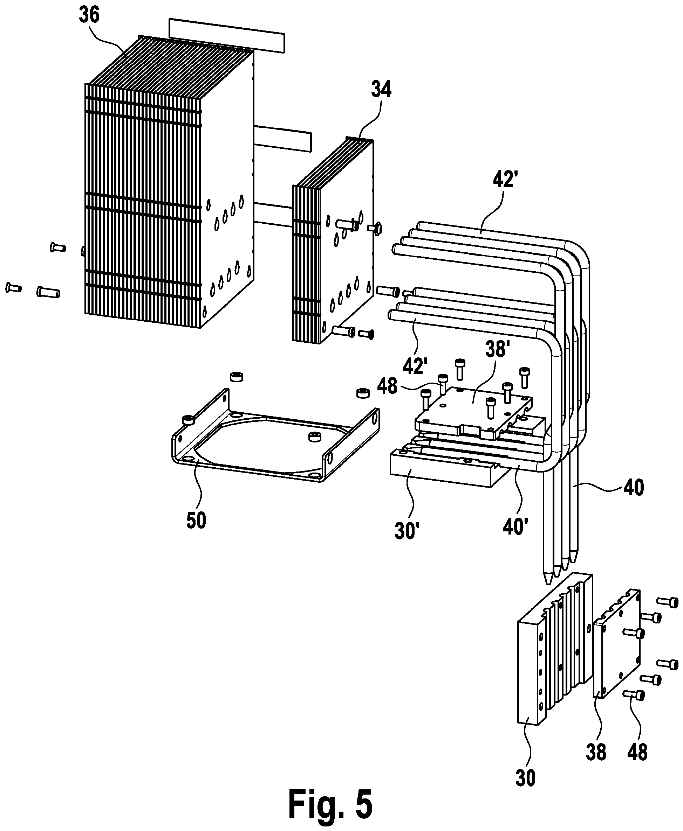

Die erste und die zweite Heatpipe 38, 38' sind vorliegend dafür ausgebildet, die thermische Energie der ersten und zweiten Wärmequelle 28, 28' zu der von den Wärmequellen 28, 28' beabstandeten Wärmesenke 34 zu führen. Hierbei mündet ein von dem ersten Endbereich 40 beabstandeter zweiter Endbereich 42 der ersten Heatpipe 32 in der Wärmesenke 34. Ein von dem ersten Endbereich 40' der zweiten Heatpipe 32' beabstandeter zweiter Endbereich 42' der zweiten Heatpipe 32' mündet vorzugsweise ebenfalls in der Wärmesenke 34 (siehe insb. Fig. 5).

Die endoskopische Beleuchtungseinrichtung 100 aus Fig. 1 weist zusätzlich zu dem Kühlkörper 36, der die Wärmesenke 34 bildet, einen Lüfter 44 auf, der dafür ausgebildet ist, einen in Richtung 45 oder in Gegenrichtung 45' des Kühlkörpers 36 weisenden Luftstrom zu erzwingen. Im vorliegenden Fall erzeugt der Lüfter 44 einen in die Richtung 45 des Kühlkörpers 36 weisenden Luftstrom. In anderen Ausführungsbeispielen ist auch ein saugend arbeitender Lüfter 44 einsetzbar, der die Luft von dem Kühlkörper 36 in die Gegenrichtung 45' absaugt. Zudem ist in Fig. 1 ein weiterer Lüfter 44 in der Peripherie des Netzteils 16 angeordnet und dafür ausgebildet, die von dem Netzteil 16, der Lichtquellen-Treiberplatine 14 und der vorzugsweise darüberliegenden Hauptplatine (nicht gezeigt) abgegebene thermische Energie aus dem Gehäuse 26 abzusaugen. In einer weiteren Ausgestaltung kann der Lüfter 44 auch derart an Kühlkörper 36 angeordnet sein, dass Luft durch den Kühlkörper ansaugt werden kann.

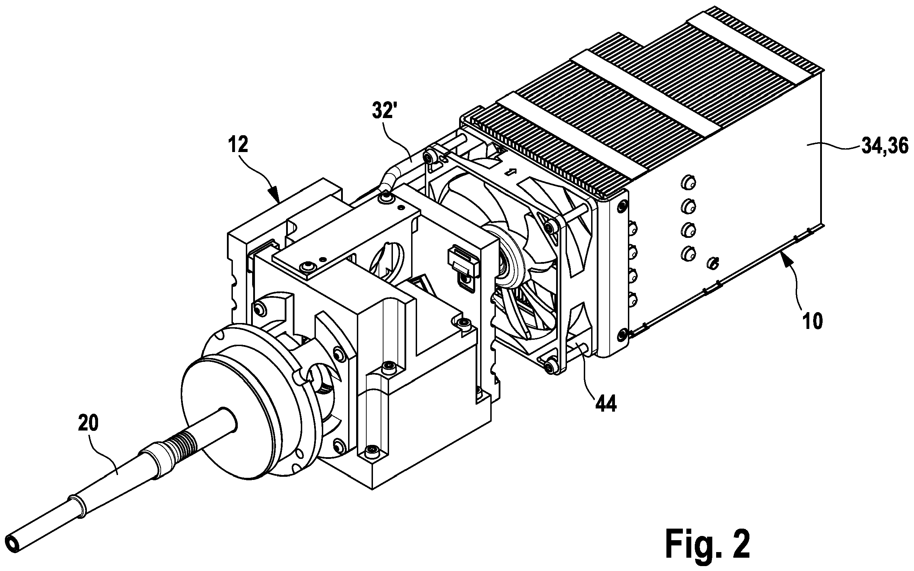

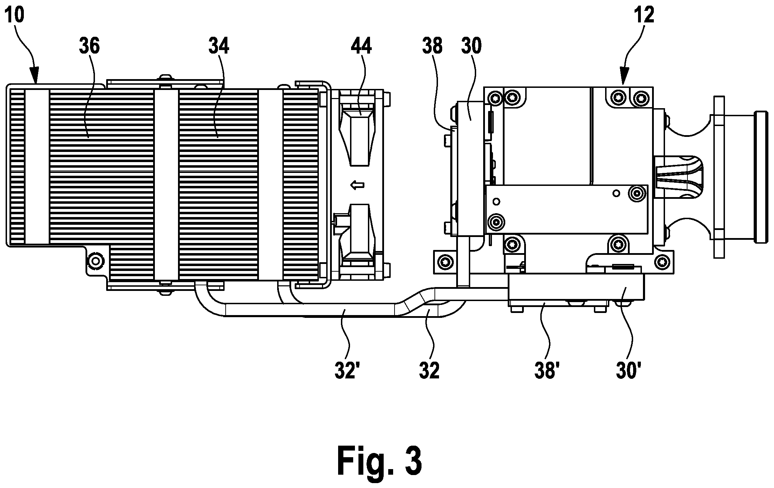

Zur besseren Übersichtlichkeit ist in den Fig. 2 und 3 die Vorrichtung 10 zusammen mit der Optikeinheit 12 nochmals isoliert, d.h. ohne die restlichen, in Fig. 1 gezeigten Komponenten der endoskopischen Beleuchtungseinrichtung 100 dargestellt.

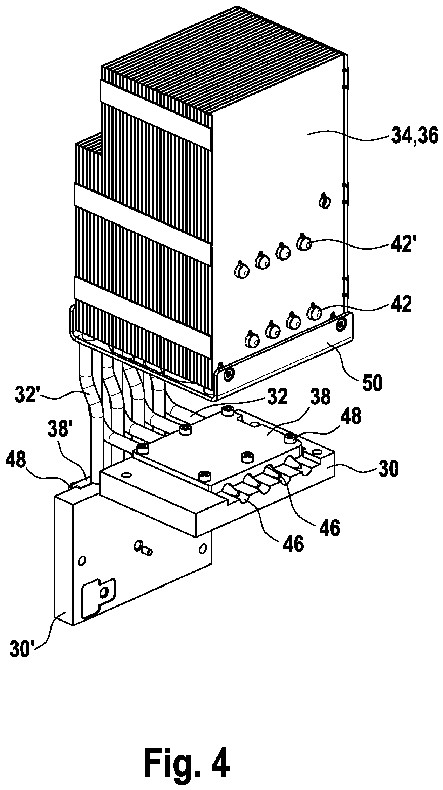

Die Fig. 4 und 5 zeigen die Vorrichtung 10 als Ausführungsbeispiel in perspektivischer Ansicht (Fig. 4) sowie in einer Explosionsansicht (Fig. 5). In Fig. 4 ist zu erkennen, dass sowohl die erste Heatpipe 32 als auch die zweite Heatpipe 32' jeweils eine Vielzahl (vorliegend jeweils vier) Wärmeleitrohre aufweisen, die von dem ersten bzw. zweiten Wärmeabfuhrelement 30, 30' zu dem Kühlkörper 36 geführt sind und in diesen münden. Dabei münden die jeweiligen zweiten Endbereiche 42, 42' der ersten und zweiten Heatpipe 32, 32' in dem Kühlkörper 36 und durchdringen diesen im vorliegenden Fall in einer Querrichtung des Kühlkörpers 36 betrachtet vollständig.

Die Wärmeabfuhrelemente 30, 30' sind vorzugsweise jeweils plattenförmig ausgestaltet und weisen mehrere Ausnehmungen 46 auf. Die Klemmelemente 38, 38' weisen vorzugsweise die gleiche Anzahl an Ausnehmungen 46 wie die Wärmeabfuhrelemente 30, 30' auf. Somit kann der erste Endbereich 40 der vorzugsweise rohrförmigen ersten Heatpipe 32 von dem ersten Wärmeabfuhrelement 30 und dem ersten Klemmelement 38 umschlossen sein. Der erste Endbereich 40' der vorzugsweise rohrförmigen zweiten Heatpipe 32' kann vorzugsweise von dem zweiten Wärmeabfuhrelement 30' und dem zweiten Klemmelement 38' umschlossen sein.

In Fig. 4 sind die Klemmelemente 38, 38' jeweils durch mehrere Schrauben 48 an dem jeweiligen Wärmeabfuhrelement 30, 30' befestigt. In anderen Ausführungsbeispielen können die Klemmelemente 38, 38' auch bspw. durch Bolzen an den Wärmeabfuhrelementen 30, 30' befestigt sein.

In der Explosionsdarstellung der Vorrichtung 10 in Fig. 5 ist zu erkennen, dass die jeweiligen ersten Endbereich 40, 40' sowie die jeweiligen zweiten Endbereiche 42, 42' der ersten bzw. zweiten Heatpipe 32, 32' jeweils geradlinig verlaufen und an einem äußeren Ende der konisch zugespitzt sind.

Die beiden Wärmeabfuhrelemente 30, 30' sind vorliegend mit mehreren Bolzen 52 an der Optikeinheit 12 (hier nicht dargestellt) montierbar. An dem Kühlkörper 36 kann vorzugsweise ein Lüfter-Montagerahmen 50 bspw. über mehrere Schrauben montiert sein, der dazu ausgebildet ist, eine Befestigungsplattform für den Lüfter 44 bereitzustellen.

Vorzugsweise kann zudem zwischen einer Oberfläche des ersten Wärmeabfuhrelements 30, einer Oberfläche des ersten Endbereichs 40 der ersten Heatpipe 32 und einer Oberfläche des ersten Klemmelements 38 eine Wärmeleitpaste zur thermischen Kopplung aufgebracht sein. In anderen Ausgestaltungen können zwischen den Lichtquellen 18, 18' und den Wärmeabfuhrelementen 30, 30' ein oder mehrere Wärmeleitpads angebracht und/oder Wärmeleitpaste aufgebracht sein.

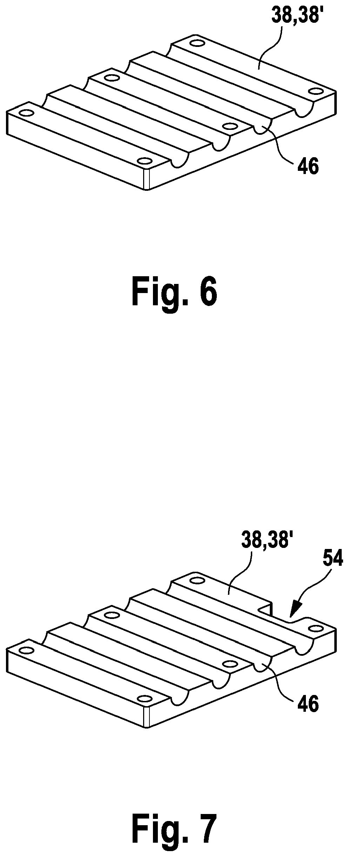

In den Fig. 6 und 7 sind zwei Ausführungsbeispiele des Klemmelements 38, 38' dargestellt, wobei sich das in Fig. 6 dargestellte Klemmelement 38 von dem in Fig. 7 dargestellten Klemmelement 38' dahingehend unterscheidet, dass es an einer Seite seiner rechteckförmigen Außenumrandung eine zusätzliche Einkerbung 54 aufweist. Die Einkerbung 54 hat keinen funktionalen Vorteil, sondern ist rein konstruktiver Natur. Wie in den Fig. 6 und 7 zu erkennen ist, wird der quaderförmige Grundkörper der Klemmelemente 38, 38' in einer Querrichtung von den mehreren halbzylinderförmigen Ausnehmungen 46 durchlaufen.

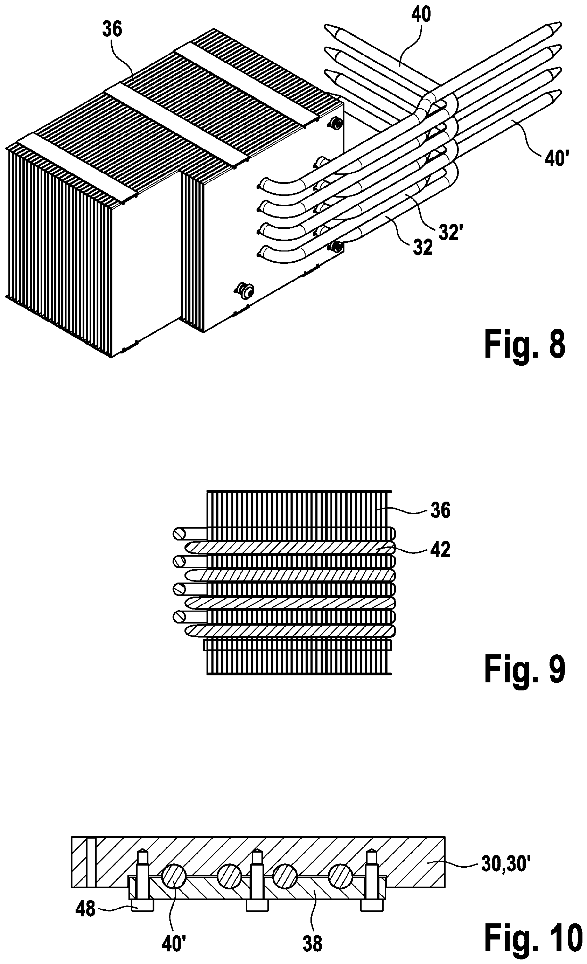

In Fig. 8 ist eine perspektivische Ansicht der Heatpipes 32, 32' in einem Zustand gezeigt, in dem die Heatpipes 32, 32' mit dem Kühlkörper 36 verlötet sind. Es ist zu erkennen, dass die ersten Endbereiche 40, 40' der Heatpipes 32, 32' freiliegen, d.h. nicht mit den Wärmeabfuhrelementen 30, 30' und den Klemmelementen 38 verklemmt sind. Fig. 9 zeigt eine Schnittansicht der in Fig. 8 gezeigten Ansicht. Es ist zu erkennen, dass die Heatpipes 32, 32' den Kühlkörper 36 in einer Querrichtung durchdringen und mit diesem verlötet sind.

Fig. 10 zeigt eine weitere Schnittansicht der Heatpipes 32, 32' in einem verklemmten / verschraubten Zustand. Hierbei sind die Heatpipes 32, 32' zwischen dem Wärmeabfuhrelement 30, 30' und dem Klemmelement 38 eingeklemmt. Die Klemmung erfolgt vorliegend durch Verschrauben mit den Schrauben 48.

## Patentansprüche

1. Vorrichtung (10) für eine endoskopische Beleuchtungseinrichtung (100), die aufweist:
- eine erste Heatpipe (32) und ein erstes Klemmelement (38);
- eine zweite Heatpipe (32') und ein zweites Klemmelement (38');- eine erste Wärmequelle (28) und eine zweite Wärmequelle (28');
- ein Wärmeabfuhrelement (30, 30') zum Abführen von thermischer Energie von der ersten Wärmequelle (28) und der zweiten Wärmequelle (28'), wobei das Wärmeabfuhrelement (30, 30') in einer direkten Peripherie der ersten Wärmequelle (28) und der zweiten Wärmequelle (28') angeordnet ist, wobei das Wärmeabfuhrelement (30, 30') eine L-Form aufweist; und- eine Wärmesenke (34), die von der ersten Wärmequelle (28) beabstandet ist,
wobei das erste Klemmelement (38) derart auf dem Wärmeabfuhrelement (30, 30') reversibel lösbar montiert ist, dass ein erster Endbereich (40) der ersten Heatpipe (32) zwischen dem Wärmeabfuhrelement (30, 30') und dem ersten Klemmelement (38) gehalten ist und das zweite Klemmelement (38') derart auf dem Wärmeabfuhrelement (30, 30') reversibel lösbar montiert ist, dass ein erster Endbereich (40') der zweiten Heatpipe (32') zwischen dem Wärmeabfuhrelement (30, 30') und dem zweiten Klemmelement (38') gehalten ist,
wobei die erste Heatpipe (32) dafür ausgebildet ist, die thermische Energie der Wärmequelle (28) zu der Wärmesenke (34) zu führen und die zweite Heatpipe (32') dafür ausgebildet ist, die thermische Energie der zweiten Wärmequelle (28') zu der Wärmesenke (34) zu führen,
wobei ein von dem ersten Endbereich (40) beabstandeter zweiter Endbereich (42) der ersten Heatpipe (32) in der Wärmesenke (34) mündet und ein von dem ersten Endbereich (40') beabstandeter zweiter Endbereich (42') der zweiten Heatpipe (32') in der Wärmesenke (34) mündet.

2. Vorrichtung nach Anspruch 1, wobei die Wärmequelle (28) eine Lichtquelle (18) ist.

3. Vorrichtung nach Anspruch 1 oder 2, wobei das erste Klemmelement (38) mit dem Wärmeabfuhrelement (30, 30') lösbar verschraubt, verstiftet oder verbolzt ist.

4. Vorrichtung nach einem der vorhergehenden Ansprüche, wobei die erste Heatpipe (32), das Wärmeabfuhrelement (30, 30') und das erste Klemmelement (38) jeweils aus einemthermisch leitfähigen Werkstoff hergestellt sind.

5. Vorrichtung nach Anspruch 4, wobei das thermisch leitfähige Material Aluminium oder Kupfer aufweist oder ist.

6. Vorrichtung nach einem der vorhergehenden Ansprüche, wobei zwischen einer Oberfläche des Wärmeabfuhrelements (30, 30'), einer Oberfläche des ersten Endbereichs (40) der ersten Heatpipe (32) und einer Oberfläche des ersten Klemmelements (38) eine Wärmeleitpaste zur thermischen Kopplung aufgebracht ist.

7. Vorrichtung nach einem der vorhergehenden Ansprüche, wobei die zweite Wärmequelle (28') eine zweite Lichtquelle (18') ist.

8. Endoskopische Beleuchtungseinrichtung (100) mit einer Vorrichtung (10) nach einem der Ansprüche 1-7.

9. Endoskopische Beleuchtungseinrichtung (100) nach Anspruch 10, die ferner aufweist:
einen Kühlkörper (36), der die Wärmesenke (34) der Vorrichtung (10) bildet; und
einen Lüfter (44), der dafür ausgebildet ist, einen in Richtung (45) zum oder in Gegenrichtung (45') weg vom Kühlkörper (36) weisenden Luftstrom zu erzwingen.

10. Endoskopische Beleuchtungseinrichtung (100) nach Anspruch 9, wobei der Kühlkörper (36) eine Vielzahl von Kühlrippen aufweist.

## Claims

1. An apparatus (10) for an endoscopic illumination device (100), comprising:
- a first heat pipe (32) and a first clamping element (38);
- a second heat pipe (32') and a second clamping element (38'); - a first heat source (28) and a second heat source (28');
- a heat dissipation element (30, 30') for dissipating thermal energy from the first heat source (28) and the second heat source (28'), the heat dissipation element (30, 30') being disposed in a direct periphery of the first heat source (28) and the second heat source (28'), the heat dissipation element (30, 30') having an L-shape; and - a heat sink (34) spaced from the first heat source (28),
wherein the first clamping element (38) is reversibly detachably mounted on the heat dissipating element (30, 30') such that a first end portion (40) of the first heat pipe (32) is held between the heat dissipating element (30, 30') and the first clamping element (38) and the second clamping element (38') is reversibly detachably mounted on the heat dissipating element (30, 30') such that a first end portion (40') of the second heat pipe (32') is held between the heat dissipating element (30, 30') and the second clamping element (38'),
wherein the first heat pipe (32) is adapted to conduct the thermal energy of the heat source (28) to the heat sink (34) and the second heat pipe (32') is adapted to conduct the thermal energy of the second heat source (28') to the heat sink (34),
wherein a second end portion (42) of the first heat pipe (32) spaced from the first end portion (40) opens into the heat sink (34) and a second end portion (42') of the second heat pipe (32') spaced from the first end portion (40') opens into the heat sink (34).

2. The apparatus according to claim 1, wherein the heat source (28) is a light source (18).

3. The apparatus according to claim 1 or 2, wherein the first clamping element (38) is detachably screwed, pinned or bolted to the heat dissipation element (30, 30').

4. The apparatus according to any one of the preceding claims, wherein the first heat pipe (32), the heat dissipation element (30, 30') and the first clamping element (38) are each made of a thermally conductive material.

5. The apparatus of claim 4, wherein the thermally conductive material comprises or is aluminum or copper.

6. The apparatus according to any one of the preceding claims, wherein a thermal paste for thermal coupling is applied between a surface of the heat dissipation element (30, 30'), a surface of the first end portion (40) of the first heat pipe (32), and a surface of the first clamping element (38).

7. The apparatus according to any one of the preceding claims, wherein the second heat source (28') is a second light source (18').

8. An endoscopic illumination device (100) comprising a device (10) according to any one of claims 1-7.

9. The endoscopic illumination device (100) of claim 10, further comprising:
a cooling element (36) forming the heat sink (34) of the device (10); and
a fan (44) adapted to force airflow in a direction (45) toward or in an opposite direction (45') away from the cooling element (36).

10. The endoscopic illumination device (100) of claim 9, wherein the cooling element (36) comprises a plurality of cooling fins.

## Revendications

1. Dispositif (10) destiné à une unité d'éclairage endoscopique (100), lequel dispositif comporte :
- un premier caloduc (32) et un premier élément de serrage (38) ;
- un deuxième caloduc (32') et un deuxième élément de serrage (38') ; - une première source de chaleur (28) et une deuxième source de chaleur (28') ;
- un élément de dissipation thermique (30, 30') destiné à dissiper l'énergie thermique de la première source de chaleur (28) et de la deuxième source de chaleur (28'), l'élément de dissipation thermique (30, 30') étant disposé dans une périphérie directe de la première source de chaleur (28) et de la deuxième source de chaleur (28'), l'élément de dissipation thermique (30, 30') ayant une forme en L ; et - un dissipateur thermique (34) qui est espacé de la première source de chaleur (28),
le premier élément de serrage (38) étant monté de manière amovible et réversible sur l'élément de dissipation thermique (30, 30') de manière à ce qu'une première zone d'extrémité (40) du premier caloduc (32) soit maintenue entre l'élément de dissipation thermique (30, 30') et le premier élément de serrage (38) et le deuxième élément de serrage (38') étant monté de manière amovible et réversible sur l'élément de dissipation thermique (30, 30') de manière à ce qu'une première zone d'extrémité (40') du deuxième caloduc (32') soit maintenue entre l'élément de dissipation thermique (30, 30') et le deuxième élément de serrage (38'),
le premier caloduc (32) étant conçu pour conduire l'énergie thermique de la source de chaleur (28) vers le dissipateur thermique (34) et le deuxième caloduc (32') étant conçu pour conduire l'énergie thermique de la deuxième source de chaleur (28') vers le dissipateur thermique (34),
une deuxième zone d'extrémité (42) du premier caloduc (32), qui est espacée de la première zone d'extrémité (40), débouchant dans le dissipateur thermique (34) et une deuxième zone d'extrémité (42') du deuxième caloduc (32'), qui est espacée de la première zone d'extrémité (40') , débouchant dans le dissipateur thermique (34).

2. Dispositif selon la revendication 1, la source de chaleur (28) étant une source de lumière (18).

3. Dispositif selon la revendication 1 ou 2, le premier élément de serrage (38) étant vissé, goupillé ou boulonné de manière amovible à l'élément de dissipation thermique (30, 30').

4. Dispositif selon l'une des revendications précédentes, le premier caloduc (32), l'élément de dissipation thermique (30, 30') et le premier élément de serrage (38) étant chacun fabriqués à partir d'un matériau thermiquement conducteur.

5. Dispositif selon la revendication 4, le matériau thermiquement conducteur étant en aluminium ou en cuivre ou comprenant ceux-ci.

6. Dispositif selon l'une des revendications précédentes, une pâte thermiquement conductrice destiné au couplage thermique étant appliquée entre une surface de l'élément de dissipation thermique (30, 30'), une surface de la première zone d'extrémité (40) du premier caloduc (32) et une surface du premier élément de serrage (38).

7. Dispositif selon l'une des revendications précédentes, la deuxième source de chaleur (28') étant une deuxième source de lumière (18').

8. Unité d'éclairage endoscopique (100) comprenant un dispositif (10) selon l'une des revendications 1 à 7.

9. Unité d'éclairage endoscopique (100) selon la revendication 10, comprenant en outre :
un corps de refroidissement (36) formant le dissipateur thermique (34) du dispositif (10) ; et
un ventilateur (44) qui est conçu pour forcer un flux d'air dans un sens (45) allant vers le corps de refroidissement (36) ou dans le sens opposé (45').

10. Unité d'éclairage endoscopique (100) selon la revendication 9, le corps de refroidissement (36) comportant un grand nombre de nervures de refroidissement.
